# EUROPEAN PATENT APPLICATION

(11) **EP 0 941 999 A2**
(43) Date of publication of application: **15.09.1999**
(21) Application number: 99301788.8
(22) Date of filing: 10.03.1999
(51) Int. Cl.: C07H 17/08

(54) **Preparation of azithromycin dihydrate**

(30) Priority: 13.03.1998 PT 10213098
(71) Applicant: HOVIONE INTER LTD., 6000 Lucerne 7 (CH)
(72) Inventor: Heggie, William, Dr., 2950 Palmela (PT); Mendez, Zita, 1000 Lisboa (PT); Bandarra, Joâo, 2670-414 Loures (PT)
(74) Representative: Wain, Christopher Paul

(57) **Abstract**

Azithromycin dihydrate is prepared from azithromycin by adding a base to an aqueous solution of azithromycin to crystallise the dihydrate, the aqueous solution having a pH of from 1 to 5 and containing acetone.

## Description

This invention relates to a process for the preparation of azithromycin dihydrate.

Azithromycin is a well gown semisynthetic macrolide antibiotic (Bright US 4,474,768; Kobrehel US 4,517,359) of formula: 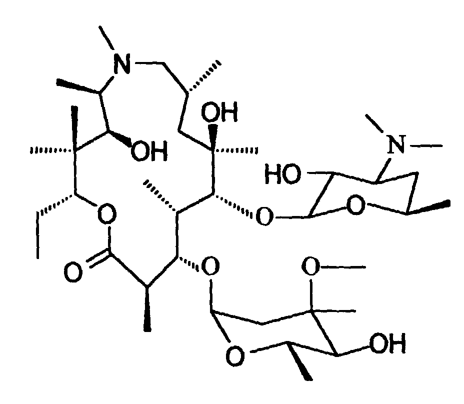 which exhibits excellent antibacterial activity against gram-positive and some gram-negative bacteria. Azithromycin is a useful therapy for infections of the upper respiratory tract in children and adults.

Azithromycin can exist in at least two distinct crystalline forms (Douglas; WO 89/00576), that are usually referred to as azithromycin monohydrate and azithromycin dihydrate. The crystal forms can be identified and differentiated by their infrared spectra, by differential scanning calorimetric thermogram and by their powder x-ray diffraction patterns.

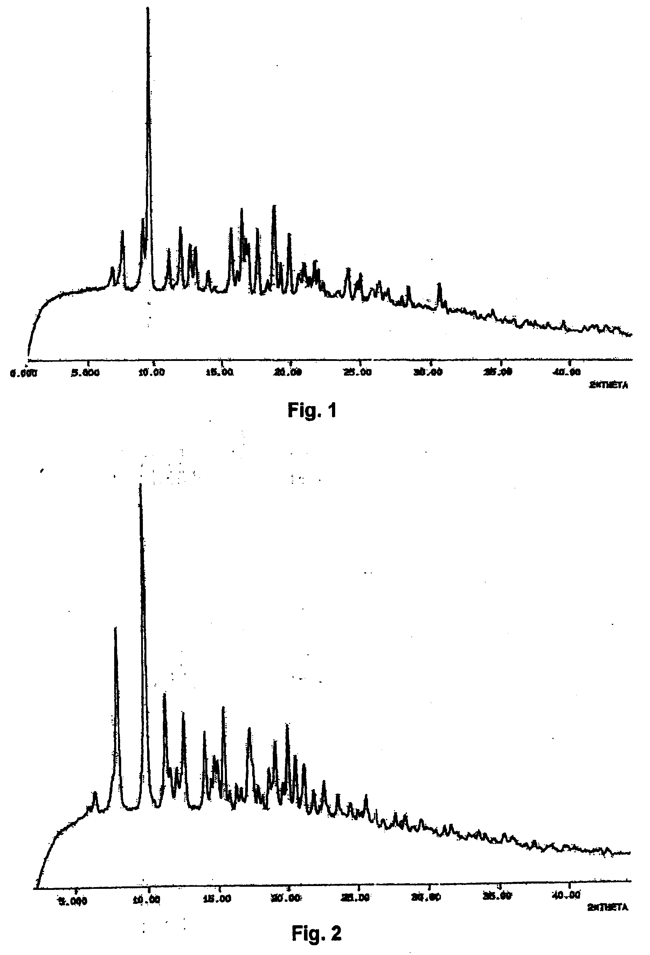

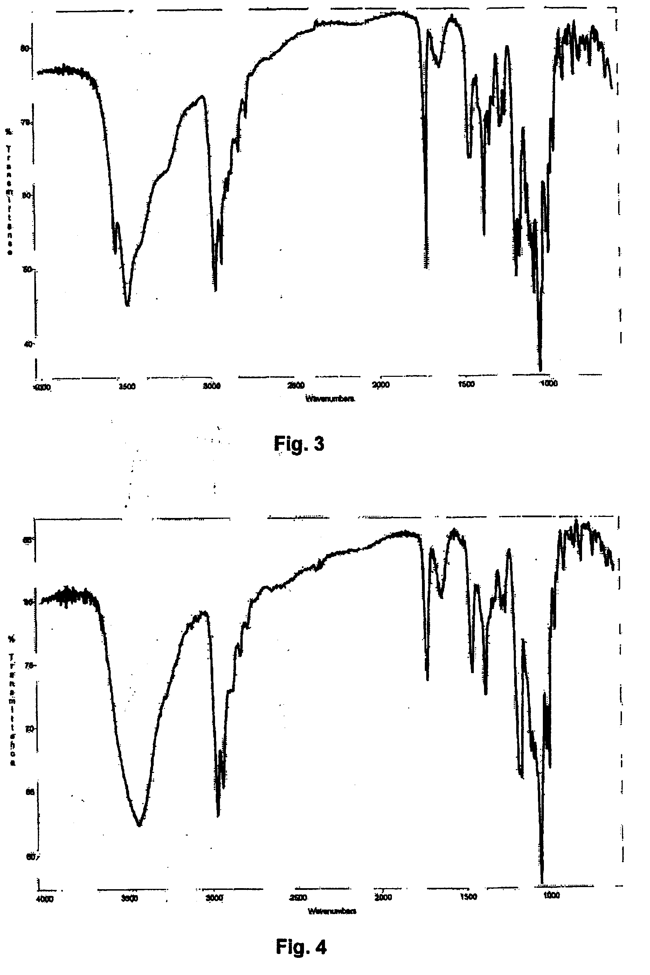

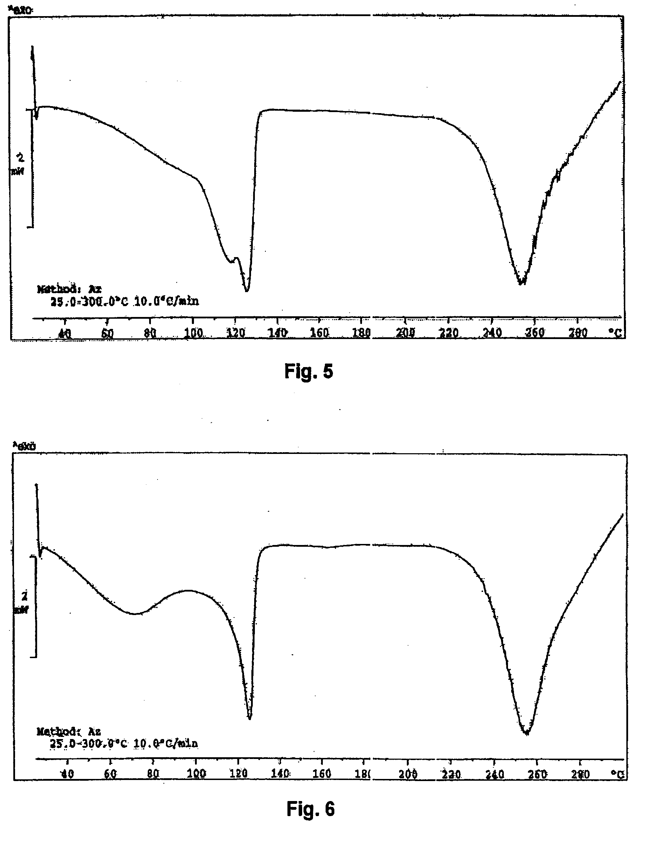

Figs. 1, 3 and 5 show, respectively, the powder x-ray diffraction spectrum, the infrared spectrum, and the differential scanning calorimetric (DSC) thermogram of azithromycin dihydrate.

Figs. 2, 4 and 6 show, respectively, the powder x-ray diffraction spectrum, the infrared spectrum, and the differential scanning calorimetric (DSC) thermogram of azithromycin monohydrate.

Drugs currently on the market are formulated from the thermodynamically more stable azithromycin dihydrate. This crystalline form has been prepared from the monohydrate by crystallising from a mixture of tetrahydrofuran (THF) and an aliphatic hydrocarbon (C₅-C₇) in the presence of at least two molar equivalents of water, as described in WO 89/00576.

However, we have noted that the preferred aliphatic hydrocarbon in WO 89/00576, hexane, whilst an excellent solvent is not otherwise ideal since it is classified in ICH class 2 due to its toxicity potential (ICH: International Conference on Harmonisation of Technical Requirements for Registration of Pharmaceuticals for Human Use). It would be advantageous to be able to manufacture azithromycin dihydrate by a process which uses less toxic solvents. In addition, the dihydrate is conventionally prepared from the isolated monohydrate and it would be advantageous if a way could be found of preparing azithromycin dihydrate directly from azithromycin itself.

We have now found a new process for the preparation of azithromycin dihydrate, which process does not require the azithromycin monohydrate form to be isolated. Furthermore, this process does not necessitate the use of solvents which have any potential toxicity problems in the production of pharmaceutical products.

According to the present invention, there is provided a process for the preparation of azithromycin dihydrate from azithromycin, which comprises adding a base to an aqueous solution of azithromycin to crystallise the dihydrate, the aqueous solution having a pH of from 1 to 5 and containing acetone.

Azithromycin can be prepared from erythromycin A. This process involves conversion of erythromycin A into its oxime; Beckmann rearrangement of the oxmie to the amino ether of erythromycin A; reduction of the amino ether to 9-deoxo-9a-aza-9a-homoerethromycin; and, finally, reductive N-methylations to obtain azithromycin.

In one procedure according to the invention, crude azithromycin is dissolved in water at a pH of 1 to 5. The low pH is obtained by addition of an acid such as dilute aqueous hydrochloric acid for example. The best results are achieved by using a volume of water of from 3 to 7 times the weight of crude azithromycin, but other volumes outside these limits can also be used. In the same way, any suitable acid can be used to set the pH but we prefer to use hydrochloric acid. The temperature is usually between 0°C and 35°C. Higher temperatures may lead to degradation of the product.

In this preferred procedure, acetone is then added to the aqueous solution of azithromycin in order to obtain a mixture wherein the weight ratio of acetone to water is from 7:3 to 3:7. A ratio of from 1:1 to 1:2 is preferred.

The pH of azithromycin solution is then adjusted to from 9 to 10 by the addition of an appropriate base. There are no limitations concerning the base which can be used but we prefer to use an aqueous solution of sodium hydroxide.

Preferably, the suspension of crystallised azithromycin is stirred for a period of from 6 to 30 hours at a temperature of from 0°C to 25°C. The time taken for this phase is critical to obtain high purity and yields of azithromycin dihydrate as initially large quantities, or even all, of the monohydrate can be precipitated at this time. Prolonged stirring serves to convert the monohydrate form completely to the dihydrate form. The exact time depends upon the temperature and composition of the solvent.

The yield is dependent on the amount of acetone present in the mixture. The yield decreases when the amount of acetone increases. When the quantity of water in the mixture is higher than 80% the azithromycin may crystallise in the form of azithromycin monohydrate.

An additional advantage of the present invention is that when azithromycin monohydrate is suspended in a mixture of water and acetone in the weight ratio of between 7:3 and 3:7 and the suspension is stirred at a temperature of between 0°C and 25°C, azithromycin dihydrate is formed. Again, the time required for complete conversion may vary from 6 to 30 hours although times outside this range may be necessary depending on the temperature.

The following non-limiting Examples illustrate the present invention.

### EXAMPLE 1

Preparation of azithromycin dihydrate. 5g of crude azithromycin, prepared as described in EP 9803945.4, was dissolved in 22.6 ml of water and 2.4 ml of hydrochloric acid (6N) at a temperature of from 20°C to 25°C. To this solution was added 25 ml of acetone and 2.8 ml of 20% (w/v) aqueous sodium hydroxide solution to adjust the pH to 9.8. After stirring for 5 hours at a temperature of from 20°C to 25°C the suspension was cooled to from 5°C to 0°C and stirred for 1 hour at this temperature. The resulting solid was collected by filtration, washed with water (3 times 5 ml of water cooled at 5°C) and dried at 35-40°C to give azithromycin dihydrate (3.3g).

### EXAMPLE 2

Preparation of azithromycin dihydrate. 5g of crude azithromycin was dissolved in 12.6 ml of water and 2.4 ml of hydrochloric acid (6N) at a temperature of from 20°C to 25°C. To this solution was added 35 ml of acetone and 2.8 ml of 20% (w/v) aqueous sodium hydroxide solution to adjust the pH to 9.8. After stirring for 5 hours at a temperature of from 20°C to 25°C the suspension was cooled to from 5°C to 0°C and stirred for 1 hour at this temperature. The resulting solid was collected by filtration, washed with water (3 times 5 ml of water cooled at 5°C) and dried at 35-40°C to give azithromycin dihydrate (2.6g).

### EXAMPLE 3

Preparation of azithromycin dihydrate. 5g of crude azithromycin was dissolved in 32.6 ml of water and 2.4 ml of hydrochloric acid (6N) at a temperature of from 20°C to 25°C. To this solution was added 15 ml of acetone and 2.8 ml of 20% (w/v) aqueous sodium hydroxide solution to adjust the pH to 9.8. After stirring for 29 hours at a temperature of from 20°C to 25°C the suspension was cooled to from 5°C to 0°C and stirred for 1 hour at this temperature. The resulting solid was collected by filtration, washed with water (3 times 5 ml of water cooled at 5°C) and dried at 35-40°C to give azithromycin dihydrate (4.6g).

### EXAMPLE 4

Preparation of azithromycin monohydrate. 5g of crude azithromycin, prepared as described in EP 9803945.4, was dissolved in 37.6 ml of water and 2.4 ml of hydrochloric acid (6N) at a temperature of from 20°C to 25°C. To this solution was added 10 ml of acetone and 2.8 ml of 20% (w/v) aqueous sodium hydroxide solution to adjust the pH to 9.8. After stirring for 4 hours at a temperature of from 20°C to 25°C the suspension was cooled to from 5°C to 0°C and stirred for 1 hour at this temperature. The resulting solid was collected by filtration, washed with water (3 times 5 ml of water cooled at 5°C) and dried at 35-40°C to give azithromycin monohydrate (4.6g).

### EXAMPLE 5

Conversion of azithromycin monohydrate to azithromycin dihydrate. Azithromycin monohydrate (5g) was suspended in 50 ml of water/acetone (30:70 by weight) and stirred for 24 hours at 20-25°C. The solid was collected by filtration, and dried at 35-40°C to give azithromycin dihydrate (4.8g).

## Claims

1. A process for the preparation of azithromycin dihydrate from azithromycin, which comprises adding a base to an aqueous solution of azithromycin to crystallise the dihydrate, the aqueous solution having a pH of from 1 to 5 and containing acetone.

2. A process according to claim 1, wherein said aqueous solution is made by dissolving azithromycin in acidified water of pH 1 to 5, and then adding acetone.

3. A process according to claim 1, wherein said aqueous solution is formed by adding an acid to a solution of azithromycin in water to provide a pH of from 1.0 to 5.0, and subsequently adding acetone.

4. A process according to claim 1, 2 or 3, wherein the pH of said aqueous solution is from 3.0 to 5.0.

5. A process according to claim 4, wherein the pH of said aqueous solution is substantially 5.

6. A process according to any of claims 1 to 5, wherein said solution contains acetic or hydrochloric acid.

7. A process according to any of claims 1 to 6, wherein said aqueous solution contains a volume of water from 3 to 7 times the weight of azithromycin.

8. A process according to any of claims 1 to 7, wherein said aqueous solution comprises crude azithromycin.

9. A process according to any of claims 1 to 8, wherein said aqueous solution comprises azithromycin monohydrate.

10. A process according to any of claims 1 to 9, wherein the weight ratio acetone: water in said aqueous solution is from 7:3 to 3:7, preferably 1:1 to 1:2.

11. A process according to any of claims 1 to 10, wherein the base is sodium hydroxide.

12. A process according to any of claims 1 to 11, wherein after addition of the base, the suspension so formed is stirred for from 6 to 30h at 0° to 25°C.

13. A process for the preparation of azithromycin dihydrate from azithromycin monohydrate, which comprises suspending the monohydrate in a mixture of water and acetone, in an acetone: water weight ratio of from 3:7 to 7:3, and stirring the suspension at 0°C to 25°C.
